# EUROPÄISCHE PATENTSCHRIFT

(11) **EP 0 915 840 B1**
(45) Veröffentlichungstag und Bekanntmachung des Hinweises auf die Patenterteilung: **15.05.2002**
(21) Anmeldenummer: 97938838.6
(22) Anmeldetag: 21.07.1997
(51) Int. Cl.: C07C 269/04, C07C 271/22

(54) **VERFAHREN ZUR HERSTELLUNG VON SUBSTITUIERTEN VALINAMID-DERIVATEN**
PROCESS FOR PREPARING SUBSTITUTED VALINE AMIDE DERIVATIVES
PROCEDE DE PREPARATION DE DERIVES D'AMIDE DE VALINE SUBSTITUES

(30) Priorität: 02.08.1996 DE 19631270
(43) Veröffentlichungstag der Anmeldung: 19.05.1999
(73) Patentinhaber: BAYER AG, 51368 Leverkusen (DE)
(72) Erfinder: RIVADENEIRA, Eric, D-51381 Leverkusen (DE)
(86) Internationale Anmeldenummer: EP9703907
(87) Internationale Veröffentlichungsnummer: WO9805633

(56) Entgegenhaltungen:
- EP-A- 0 472 996
- M. R. VERNSTEN ET AL.: "The Preparation of Some N-Carbethoxyamino Acids" JOURNAL OF THE AMERICAN CHEMICAL SOCIETY, Bd. 75, Nr. 6, März 1953, DC US, Seiten 1320-1321, XP002046973

## Beschreibung

Die Erfindung betrifft ein verfahren zur Herstellung von Valinamid-Derivaten.

Die Valinamid-Derivate sind bekannt und besitzen eine ausgezeichnete Wirkung bei der Bekämpfung von Schädlingen. Insbesondere können sie als Fungizide, vor allem im Pflanzenschutz verwendet werden (EP-472 996).

Es wurde nun gefunden, daß man die bekannten Valinamid-Derivate einfacher und mit höherer Ausbeute sowie Reinheit herstellen kann, wobei im wesentlichen auf die Verwendung organischer Lösungsmittel verzichtet werden kann.

Gegenstand der Anmeldung ist daher ein Verfahren zur Herstellung von Verbindungen der Formel (I) 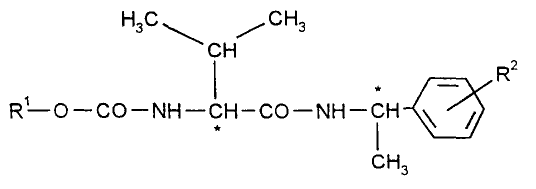 in welcher
- R¹: für i-Propyl und
- R²: für Chlor, Methyl, Ethyl oder Methoxy steht,
indem man
a) eine wäßrige alkalische Lösung von L-Valin mit Chlorameisensäureisopropylester reagieren läßt und die so erhaltenen Reaktionsprodukte nach Neutralisation
b) nochmals, gegebenenfalls in Gegenwart organischer Lösungsmittel, in Gegenwart katalytischer Mengen eines tertiären Amins mit Chlorameisensäureisopropylester reagieren läßt und anschließend die so erhaltenen Reaktionsprodukte, gegebenenfalls nach Versetzen mit organischen Lösungsmitteln
c) mit in organischen Lösungsmitteln gelösten, entsprechend substituierten Phenethylamin reagieren läßt.

Die nach diesem Verfahren erhältlichen Valinamid-Derivate sind gut aufzuarbeiten und zu isolieren und fallen in kompakter Form mit großer Raum/Zeit Ausbeute an.

Das erfindungsgemäße Verfahren bzw. die einzelnen Verfahrensschritte a) und b) werden vorzugsweise in Wasser als einzigem Lösungs- bzw. Verdünnungsmittel durchgeführt.

Die Produkte der Verfahrensschritte a) und b) können dabei isoliert und für die weiteren Reaktionen eingesetzt werden. Vorzugsweise wird das Verfahren aber ohne Zwischenisolierung als sogenanntes Eintopfverfahren, gegebenenfalls unter Rückführung der Mutterlauge in einen weiteren Ansatz oder gegebenenfalls mit kontinuierlicher Verfahrensführung, durchgeführt.

Für die Verfahrensschritte a) und b) wird vorzugsweise Chlorameisensäureisopropylester eingesetzt. Der Ester wird dabei vorzugsweise zu den entsprechenden Lösungen bzw. Reaktionsmischungen gegeben, wobei der Ester vorzugsweise im molaren Überschuß, bezogen auf Valin bzw das Reaktionsprodukt aus Verfahrensschritt a) und insgesamt in Mengen von 2 bis 3 mol, bezogen auf 1 mol Valin zugegeben wird.

Die Zugabe- bzw. Reaktionstemperaturen für Verfahrensschritt a) liegen dabei vorzugsweise bei -20 bis 80°C, insbesondere bei 10 bis 50°C und für Verfahrensschritt b) vorzugsweise bei -20 bis 40°C, insbesondere 10 bis 30°C. Bevorzugt werden die Verfahrensschritte a) und b) bei Raumtemperatur gegebenenfalls unter Kühlung der exotherm ablaufenden Reaktion durchgeführt.

Der Verfahrensschritt a) wird im alkalischen Medium, d.h. vorzugsweise in Gegenwart anorganischer Basen wie KOH und insbesondere NaOH durchgeführt. Die Base liegt im Überschuß, insbesondere in einer Menge von 1 bis 3 mol, bezogen auf 1 mol Valin, vor.

Die Neutralisation in Verfahrensschritt a) erfolgt vorzugsweise mit anorganischen Säuren wie Schwefelsäure und insbesondere Salzsäure, insbesondere auf einem pH-Wert von ca. 7.

Der Verfahrensschritt b) wird in Gegenwart katalytischer Mengen eines tertiären Amines, vorzugsweise Pyridin, Methylpyridin, Dimethylaminopyridin, Triethylamin und/oder insbesondere Dimethylbenzylamin (Desmorapid DB®) durchgeführt. Unter katalytischen Mengen werden in der vorliegenden Anmeldung vorzugsweise Mengen von 1/10 000 bis 1/100 mol Katalysator pro mol Valin verstanden.

Von besonderer Bedeutung für den Verfahrensschritt c) bzw. für die spätere Aufarbeitung ist, daß die Phenethylamine in wenig organischem Lösungsmittel gelöst und erst dann mit den Reaktionsprodukten aus Verfahrensschritt b) zur Reaktion gebracht werden.

Als Lösungsmittel werden vorzugsweise Essigsäureester, Methyl-tert.-butylether und insbesondere tert.-Amyl-methylether (TAME) eingesetzt. Die Menge an eingesetztem organischen Lösungsmittel beträgt dabei vorzugsweise 50 bis 800 ml und insbesondere 100 bis 500 ml, bezogen auf 0,3 mol des jeweiligen Phenethylamins. Die dermaßen vorgelösten Phenethylamine werden dann vorzugsweise zu der Reaktionsmischung aus Verfahrensschritt b) gegeben, wobei die Menge an Phenethylamin äquimolar bzw. im geringen Überschuß zu Valin ist.

Zur besseren Aufarbeitung der in Verfahrensschritt c) anfallenden Zielverbindungen werden die Reaktionsmischungen aus Verfahrensschritt c), gegebenenfalls unter Druck auf Temperaturen von 40 bis zur Siedetemperatur der jeweiligen organischen Lösungsmittel erhitzt, bis daß eine optimale Trennung der organischen Phase von der wäßrigen Phase zu beobachten ist. Die Aufarbeitung der organischen Phase erfolgt dann nach üblichen Methoden.

Die nach dem erfindungsgemäßen Verfahren erhältlichen Produkte, die im wesentlichen aus definierten Isomerengemischen der gewünschten Valinamid-Derivate bestehen, können ohne weitere Reinigung z.B. als Fungizide im Pflanzenschutz eingesetzt werden.

Das erfindungsgemäße Verfahren hat somit neben den oben genannten Vorzügen die Vorteile, daß das Verfahren in Wasser als Lösungs- und Verdünnungsmittel, vorzugsweise 50 bis 500 g, insbesondere 100 bis 300 g Wasser, die gewünschten Produkte in hoher Ausbeute und hoher Reinheit anfallen. Die nachfolgenden Beispiele dienen zur Erläuterung der Erfindung. Die Erfindung ist nicht auf die Beispiele beschränkt.

### Beispiele

### Beispiel 1

Zu einem Gemisch aus 105 g Wasser und 63,6 g (0,716 mol) 45 %iger Natronlauge werden bei Raumtemperatur 35,8 g (0,305 mol) L-Valin gegeben. Man rührt, bis eine klare Lösung vorliegt.

Innerhalb von 2 Stunden werden anschließend bei Raumtemperatur 45,4 g (0,366 mol) Chlorameisensäureisopropylester zugetropft. Man läßt 30 Minuten nachrühren. Mit 37 %iger Salzsäure wird ein pH-Wert von 7 eingestellt.

Anschließend werden 0,4 g (0,003 mol) Desmorapid DB® zum Reaktionsgemisch gegeben. Man läßt 15 Minuten nachrühren und setzt anschließend 38,4 g (0,31 mol) Chlorameisensäureisopropylester zu. Es wird eine weitere Stunde nachgerührt.

In zwei Stunden wird eine Lösung von 44,6 g (0,31 mol) p-Methylphenethylamin (3) in 200 ml TAME (tert.-Amyl-methylether) zudosiert. Es fällt sofort ein weißer Feststoff aus, das Gemisch bleibt jedoch rührbar.

Anschließend wird auf 70°C erwärmt, bei dieser Temperatur die Phasentrennung durchgeführt und die organische Phase mit 100 ml Wasser nachgewaschen. Man läßt auf 40°C abkühlen und filtriert bei dieser Temperatur ab. Der Nutschkuchen wird mit wenig TAME nachgewaschen und anschließend getrocknet.

Man erhält 78,4 g des Produktes (Gehalt: 96,3 %). Dies entspricht einer Ausbeute von 77,3 % d. Th.

Aus der organischen Mutterlauge erhält man nach Abdestillieren des Lösungsmittels 35,1 g eines Rückstandes, in dem das gewünschte Produkt zu 43,7 % enthalten ist (Ausbeute: 15,7 % d. Th.).

### Vergleichsbeispiel 2

Zu einem Gemisch aus 105 g Wasser und 63,6 g (0,716 mol) 45 %iger Natronlauge werden bei Raumtemperatur 35,8 g (0,305 mol) L-Valin gegeben. Man rührt, bis eine klare Lösung vorliegt.

Innerhalb von 2 Stunden werden anschließend bei Raumtemperatur 45,4 g (0,366 mol) Chlorameisensäureisopropylester zugetropft. Man läßt 30 Minuten nachrühren. Mit 37 %iger Salzsäure wird ein pH-Wert von 7 eingestellt.

Anschließend werden 0,4 g (0,003 mol) Desmorapid DB zum Reaktionsgemisch gegeben. Man läßt 15 Minuten nachrühren und setzt anschließend 38,4 g (0,31 mol) Chlorameisensäureisopropylester zu. Es wird eine weitere Stunde nachgerührt.

In zwei Stunden werden 44,6 (0,31 mol) p-Methylphenethylamin (3) zudosiert. Man läßt eine Stunde nachrühren. Während dieser Zeit wird die gebildete Suspension sehr dick, bleibt aber noch gerade rührbar. Das Gemisch wird anschließend bei Raumtemperatur abgesaugt und der Nutschkuchen mit 500 ml Wasser nachgewaschen. Nach Trocknung erhält man 99,5 g eines Feststoffes, der das gewünschte Produkt zu 65,1 % enthält. Dies entspricht einer Ausbeute von 66,3 % d. Th.

### Beispiel 3

35,8 g (0,305 mol) L-Valin werden bei Raumtemperatur in einem Gemisch aus 105 g Wasser und 63,6 g (0,716 mol) 45 %iger Natronlauge gelöst. Es wird solange gerührt, bis eine klare Lösung vorliegt.

Anschließend werden innerhalb 2 Stunden 45,4 g (0,366 mol) Chlorameisensäureisopropylester (98,9 %ig) zugetropft. Man läßt 30 Minuten nachrühren und stellt durch Zugabe von 2,9 g 37 %iger Salzsäure einen pH-Wert von 7 ein.

Es werden 650 ml TAME und 0,4 g Desmorapid DB® zum Reaktionsmedium gegeben. Es wird weitere 15 Minuten nachgerührt und anschließend innerhalb einer Stunde mit 38,4 g (0,31 mol) Chlorameisensäureisopropylester (98,9 %ig) versetzt.

Man läßt eine Stunde nachrühren und dosiert nun in 2 Stunden eine Lösung aus 44,6 g (0,31 mol) p-Methylphenethylamin (93,9 %ig) in 130 ml TAME zu. Man läßt eine Stunde nachrühren und gibt 100 ml Natronlauge (IN) zurSuspension. Bei 55°C werden die Phasen getrennt. Die organische Phase wird bei 55°C einmal mit 50 ml Wasser extrahiert und anschließend auf 0°C abgekühlt.

Man rührt 30 Minuten nach und saugt den Feststoff im Vakuum ab. Der Nutschkuchen wird mit etwa TAME nachgewaschen und im Vakuumtrockenschrank getrocknet. Das Filtrat wird am Rotationsverdampfer eingeengt.

Es werden 75,1 g eines Nutschkuchens erhalten, der einen Wirkstoffgehalt von 99,7 % aufweist (76,6 % d.Th.).

Der eingeengte Mutterlaugenrückstand (17,2 g) weist einen Wirkstoffgehalt von 56,6 % auf.

### Beispiel 4

35,8 g (0,305 mol) L-Valin werden bei Raumtemperatur in einem Gemisch aus 105 g Wasser und 63,6 g (0,716 mol) 45 %iger Natronlauge gelöst. Es wird solange gerührt, bis eine klare Lösung vorliegt.

Anschließend werden innerhalb 2 Stunden 45,4 g (0,366 mol) Chlorameisensäureisopropylester (98,9 %ig) zugetropft. Man läßt 30 Minuten nachrühren und stellt durch Zugabe von 3 g 37 %iger Salzsäure einen pH-Wert von 7 ein.

Es werden 650 ml TAME, 15,3 g des Mutterlaugenrückstandes aus Beispiel 3 und 0,4 g Desmorapid DB® zum Reaktionsmedium gegeben.

Es wird weitere 15 Minuten nachgerührt und anschließend innerhalb einer Stunde mit 38,4 g (0,31 mol) Chlorameisensäureisopropylester (98,9 %ig) versetzt. Man läßt eine Stunde nachrühren und dosiert nun in 2 Stunden eine Lösung aus 44,6 g (0,31 mol) p-Methylphenethylamin (93,9 %ig) in 130 ml TAME zu. Man läßt eine Stunde nachrühren und gibt 100 ml Natronlauge (IN) zur Suspension. Bei 55°C werden die Phasen getrennt. Die organische Phase wird bei 55°C einmal mit 100 ml Wasser extrahiert und anschließend auf 0°C abgekühlt. Man rührt 30 Minuten nach und saugt den Feststoff im Vakuum ab. Der Nutschkuchen wird mit etwas TAME nachgewaschen und im Vakuumtrockenschrank getrocknet. Das Filtrat wird am Rotationsverdampfer eingeengt.

Es werden 92,8 g eines Nutschkuchens erhalten, der einen Wirkstoffgehalt von 97,4 % aufweist (92,5 % d.Th.).

## Patentansprüche

1. Verfahren zur Herstellung von Verbindungen der Formel (I) 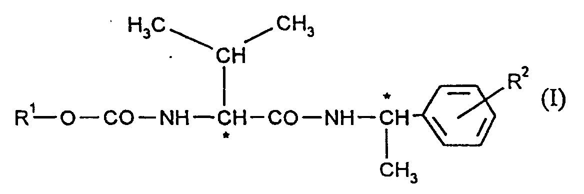 in welcher
R¹ für i-Propyl und
R² für Chlor, Methyl, Ethyl oder Methoxy steht,
**dadurch gekennzeichnet, daß** man
a) eine wäßrige alkalische Lösung von L-Valin mit Chlorameisensäureisopropylester reagieren läßt und die so erhaltenen Reaktionsprodukte nach Neutralisation
b) nochmals in Gegenwart katalytischer Mengen eines tert. Amins und gegebenenfalls in Gegenwart organischer Lösungsmittel mit Chlorameisensäureisopropylester reagieren läßt und anschließend die so erhaltenen Reaktionsprodukte gegebenenfalls mit organischen Lösungsmitteln versetzt und anschließend
c) mit in organischen Lösungsmitteln gelösten, entsprechend substituierten Phenethylamin reagieren läßt.

2. Verfahren gemäß Anspruch 1, **dadurch gekennzeichnet, daß** die Zugabebzw. Reaktionstemperatur des Verfahrensschritts a) bei -20 bis 80°C, der Verfahrensschritt b) bei -20 bis 40°C und der Verfahrensschritt c) bei 0 bis 30°C liegt.

3. Verfahren gemäß Anspruch 1, **dadurch gekennzeichnet, daß** als katalytisch wirkende tert. Amine im Verfahrensschritt b) Pyridin, Methylpyridin, Dimethylaminopyridin, Triethylamin und/oder Dimethylbenzylamin eingesetzt werden.

4. Verfahren gemäß Anspruch 1, **dadurch gekennzeichnet, daß** als organisches Lösungsmittel Essigsäureester, Methyl-tert.-butylether oder tert.-Amylmethylether eingesetzt werden.

5. Verfahren gemäß Anspruch 1, **dadurch gekennzeichnet, daß** die Menge des organischen Lösungsmittel 50 bis 800 ml, bezogen auf 0,3 mol des jeweiligen Phenethylamines beträgt.

6. Verfahren gemäß Anspruch 1, **dadurch gekennzeichnet, daß** im Verfahrensschritt c) das Reaktionsgemisch nach der Zugabe des Phenethylamins auf 40°C bis zur Siedetemperatur des jeweiligen organischen Lösungsmittels erhitzt wird.

7. Verfahren gemäß Anspruch 1, **dadurch gekennzeichnet, daß** in Verfahrensschritt c) p-Methylphenethylamin eingesetzt wird.

## Claims

1. Process for the preparation of compounds of the formula (I) 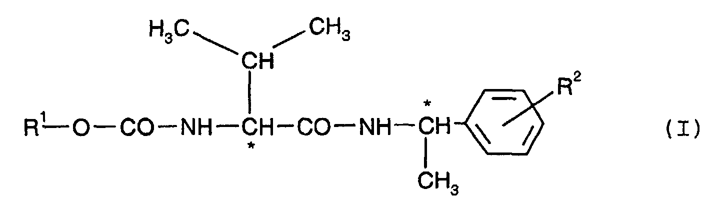 in which
R¹ is i-propyl, and
R² is chlorine, methyl, ethyl or methoxy,
**characterized in that**
a) an aqueous alkaline solution of L-valine is reacted with isopropyl chloroformate, and the resulting reaction products, after neutralization,
b) are again reacted with isopropyl chloroformate in the presence of catalytic amounts of a tertiary amine and optionally in the presence of organic solvents, and then the resulting reaction products are optionally admixed with organic solvents and then
c) reacted with correspondingly substituted phenethylamine dissolved in organic solvent.

2. Process according to Claim 1, **characterized in that** the addition or reaction temperature of process step a) is from -20 to 80°C, process step b) is from -20 to 40°C and process step c) is from 0 to 30°C.

3. Process according to Claim 1, **characterized in that** the catalytically active tert. amines in process step b) are pyridine, methylpyridine, dimethylaminopyridine, triethylamine and/or dimethylbenzylamine.

4. Process according to Claim 1, **characterized in that** the organic solvent is acetic ester, methyl tert-butyl ether or tert-amyl methyl ether.

5. Process according to Claim 1, **characterized in that** the amount of organic solvent is from 50 to 800 ml, based on 0.3 mol of the respective phenethylamine.

6. Process according to Claim 1, **characterized in that** in process step c), after phenethylamine has been added, the reaction mixture is heated to from 40°C to the boiling temperature of the respective organic solvent.

7. Process according to Claim 1, **characterized in that** in process step c) p-methylphenethylamine is used.

## Revendications

1. Procédé de préparation de composés de la formule (I) : 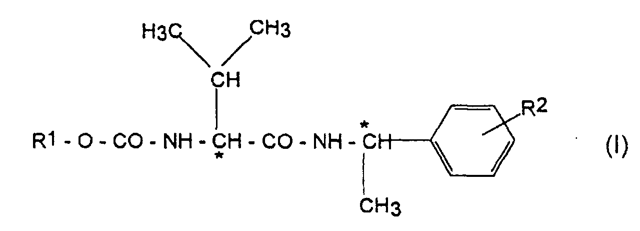 dans laquelle :
R¹ représente le radical i-propyle, et
R² représente l'atome de chlore, les radicaux méthyle, éthyle ou méthoxy,
**caractérisé en ce que**
a) on fait réagir une solution aqueuse alcaline de L-valine avec l'ester isopropylique de l'acide chloroformique et le produit de réaction ainsi obtenu, après neutralisation,
b) est encore une fois mis à réagir avec l'ester isopropylique de l'acide chloroformique, en présence d'une quantité catalytique d'une amine tertiaire et le cas échéant en présence d'un solvant organique, et ensuite, on ajoute au produit de réaction ainsi obtenu, le cas échéant un solvant organique, et ensuite
c) on le fait réagir avec une phénéthylamine substituée appropriée, dissoute dans un solvant organique.

2. Procédé suivant la revendication 1, **caractérisé en ce que** la température d'addition ou de réaction de l'étape a) du procédé se situe dans l'intervalle allant de -20 à 80°C, de l'étape b) du procédé dans l'intervalle allant de -20 à 40°C, et de l'étape c) du procédé dans l'intervalle allant de 0 à 30°C.

3. Procédé suivant la revendication 1, **caractérisé en ce que** l'on met en oeuvre comme amine tertiaire catalytique dans l'étape b) du procédé, la pyridine, la méthylpyridine, la diméthylaminopyridine, la triéthylamine et/ou la diméthylbenzylamine.

4. Procédé suivant la revendication 1, **caractérisé en ce que** l'on met en oeuvre comme solvant organique, un acétate, le méthyl-t-butyléther ou le t-amylméthyléther.

5. Procédé suivant la revendication 1, **caractérisé en ce que** la quantité de solvant organique se situe dans l'intervalle allant de 50 à 800 ml, par rapport à 0,3 mole de la phénéthylamine choisie.

6. Procédé suivant la revendication 1, **caractérisé en ce que** dans l'étape c) du procédé, on chauffe le mélange réactionnel après addition de la phénéthylamine, de 40°C à la température d'ébullition du solvant organique.

7. Procédé suivant la revendication 1, **caractérisé en ce que** l'on met en oeuvre dans l'étape c) du procédé, la p-méthylphénéthylamine.
